# EUROPÄISCHE PATENTSCHRIFT

(11) **EP 2 413 138 B1**
(45) Veröffentlichungstag und Bekanntmachung des Hinweises auf die Patenterteilung: **29.04.2020**
(21) Anmeldenummer: 10015721.3
(22) Anmeldetag: 16.12.2010
(51) Int. Cl.: G01N 33/49, G01N 33/80, B01L 3/00

(54) **VORRICHTUNG UND VERFAHREN ZUR ABTRENNUNG VON BESTANDTEILEN EINER PROBENFLÜSSIGKEIT**
DEVICE AND METHOD FOR SEPARATING COMPONENTS OF A LIQUID SAMPLE
DISPOSITIF ET PROCÉDÉ DE SÉPARATION DE COMPOSANTS D'UN LIQUIDE D'ÉCHANTILLON

(30) Priorität: 27.07.2010 EP 10007766
(43) Veröffentlichungstag der Anmeldung: 01.02.2012
(73) Patentinhaber: BOEHRINGER INGELHEIM microParts GmbH, 44227 Dortmund (DE)
(72) Erfinder: BLANKENSTEIN, Gert, 55216 Ingelheim am Rhein (DE); RODENFELS, Tobias, 55216 Ingelheim am Rhein (DE)
(74) Vertreter: Von Rohr Patentanwälte Partnerschaft mbB

(56) Entgegenhaltungen:
- EP-A2- 0 260 965
- EP-A2- 0 373 863
- WO-A1-97/23780
- WO-A1-2007/099344
- WO-A2-2007/057744
- WO-A2-2009/106331
- FR-A1- 2 929 135
- US-A- 3 799 742
- US-A- 6 019 944
- US-A1- 2006 105 469

## Beschreibung

Die vorliegende Erfindung betrifft eine Vorrichtung zur Abtrennung von Bestandteilen einer Probenflüssigkeit, insbesondere Blut, gemäß dem Oberbegriff des Anspruchs 1 sowie ein derartiges Verfahren.

Die vorliegende Erfindung befasst sich mit mikrofluidischen Systemen bzw. Vorrichtungen. Die nachfolgenden Ausführungen beziehen sich daher bevorzugt auf Vorrichtungen und Verfahren, bei denen Kapillarkräfte wirken und insbesondere für die Funktion entscheidend sind. Vorzugsweise ist die vorliegende Erfindung aber auch bei sonstigen, insbesondere druckbetriebenen Vorrichtungen und Verfahren einsetzbar.

Eine mikrofluidische Vorrichtung zur Plasmaseparation ist beispielsweise aus der WO 2009/106331 A2 bekannt. Die Vorrichtung weist eine Zuführeinrichtung zur Aufnahme des Blutes, eine Trenneinrichtung zur Abtrennung von Blutbestandteilen und eine Struktur zur Ableitung aus. In der Zuführeinrichtung kann eine Trockenchemikalie vorgesehen sein. Weiterhin offenbart die WO 2009/106331 A2 eine Aufnahme mit einer Membran als Trenneinrichtung. Die Membran kann durch einen Stößel gewölbt werden, um einem Flüssigkeitsabfließen in einen Kanal durch Kapillarkräfte zu induzieren.

Die WO 97/23780 A1 betrifft Testeinrichtungen zur Plasmaseparation. Diese weisen einen klappbaren Aufnahmebereich auf, der mit einer Trenneinrichtung verbunden werden kann. Der Aufnahmebereich kann porös und mit einer Trockenchemikalie beschichtet sein.

Die US 6,019,944 A betrifft Vorrichtungen zur Diagnose und Mittel zur kontrollierten Bewegung von Reagenzien. Es werden mikrostrukturierte Flächen zur Erzeugung von Kapillar- bzw. Haltekräften offenbart.

Die FR 2 929 135 A1 betrifft eine Trenneinrichtung für Blutbestandteile. Die Einrichtung weist eine Aufnahme, einen Kanal, eine Trenneinrichtung und eine Struktur zur Ableitung der Flüssigkeit auf.

Die WO 2007/099344 A1 betrifft eine klappbare Trennvorrichtung für Blutbestandteile. Die Vorrichtung weist eine Aufnahmeeinrichtung, eine Trenneinrichtung und eine nachgeordnete Struktur zur Ableitung einer Probe auf. Weiterhin weist die Einrichtung ein Leitelement auf, zur Herstellung einer fluidischen Verbindung zwischen einem Aufnahmebereich durch einen Zwischenbereich und Trennbereich.

Die US 3 799 742 A betrifft eine Trenneinrichtung für Blutbestandteile. Die Einrichtung weist eine Aufnahmeeinrichtung, eine Trenneinrichtung und eine nachgeordnete Struktur zur Ableitung einer Probe auf. In einer Ausführungsform weist die Aufnahmeeinrichtung durch Trennwände getrennte und hintereinander lie-gende Aufnahmekammern auf, die durch ein Anstechelement geöffnet und fluidisch mit einem Filterelement verbunden werden.

Die EP 0 260 965 A2 offenbart eine Vorrichtung zur Immundiagnostik. Die Vorrichtung umfasst eine Filtereinrichtung, die über einem Matrixmaterial angeordnet ist, so dass Flüssigkeit durch die Filtereinrichtung gefiltert und dem Matrixmaterial zugeführt wird. Das Matrixmaterial bindet Antikörper der Flüssigkeit zur immundiagnostischen Untersuchung.

Die EP 0 373 863 A2 offenbart eine Vorrichtung zur Bestimmung eines Analyten in einer Flüssigkeitsprobe. Die Vorrichtung hat eine Abdeckung mit einer Aufnahme, die eine Durchlassöffnung aufweist. Die Vorrichtung ist dabei so ausgebildet, dass Flüssigkeit aufgrund von Oberflächenspannung in der Aufnahme gehalten wird ohne durch die Durchlassöffnung zu fließen. Durch Drücken auf einen Umfangsbereich der Aufnahme kann diese mit einem darunter befindlichen porösen Element in Kontakt gebracht werden, sodass die Flüssigkeit aufgrund von Kapillarkräften durch die Durchlassöffnung in das poröse Element gesaugt wird. Im porösen Element reagiert der Analyt der Flüssigkeit und erzeugt so ein messbares Signal.

Der vorliegenden Erfindung liegt die Aufgabe zu Grunde, eine Vorrichtung und ein Verfahren zur Abtrennung von Bestandteilen einer Probenflüssigkeit anzugeben, wobei eine einfache Vorbehandlung der Probenflüssigkeit durch mindestens eine Chemikalie in der Vorrichtung vor Kontakt mit einer Trenneinrichtung und/oder eine einfache Dosierung der zuzuführenden Probenflüssigkeit ermöglicht.

Die obige Aufgabe wird durch eine Vorrichtung gemäß Anspruch 1 oder ein Verfahren gemäß Anspruch 5 gelöst. Vorteilhafte Weiterbildungen sind Gegenstand der Unteransprüche.

Eine vorschlagsgemäße Vorrichtung weist eine Aufnahmeeinrichtung auf, die ihrerseits einen Aufnahmebereich zum Anhalten oder Verzögern der Probenflüssigkeit durch Kapillarkraft und bevorzugt eine von der Probenflüssigkeit lösbare Chemikalie zur Vorbehandlung der Probenflüssigkeit vor Zuführung zu einer Trenneinrichtung aufweist. Besonders bevorzugt handelt es sich um eine im Aufnahmebereich eingetrocknete Chemikalie, die von der Probenflüssigkeit vor Zuführung zur Trenneinrichtung gelöst wird. Der Aufnahmebereich ist dabei vorzugsweise zum Anhalten oder Verzögern der Probenflüssigkeit durch Kapillarkraft ausgebildet. Der Aufnahmebereich ist insbesondere auch bei druckbetriebenen Systemen, Vorrichtungen oder Verfahren einsetzbar. So kann auf sehr einfache Weise eine Vorbehandlung der Probenflüssigkeit durch die Chemikalie vor der eigentlichen Abtrennung erfolgen. Insbesondere kann die Vorbehandlung definierter erfolgen, als dies der Fall ist, wenn die Chemikalie in die Trenneinrichtung bzw, ein Filterelement der Trenneinrichtung integriert wäre.

Besonders bevorzugt weist die Aufnahmeeinrichtung bzw. deren Aufnahmebereich eine schräge Rampe mit einer zugeordneten Abdeckung oder eine Vielzahl von Erhebungen, die insbesondere ein Säulenfeld bilden, auf. Dies gestattet auf sehr einfache Weise das gewünschte Dosieren, Anhalten oder Verzögern der Probenflüssigkeit durch Kapillarkraft.

Das vorschlagsgemäße Verfahren zeichnet sich insbesondere dadurch aus, dass die Probenflüssigkeit in die mikrofluidische Vorrichtung aufgenommen wird und dort in einem Aufhahmebereich der Vorrichtung getrennt von der Trenneinrichtung mit einer Chemikalie vorbehandelt wird und erst nach der Vorbehandlung weiter zur Trenneinrichtung zur Abtrennung von Bestandteilen aus der vorbehandelten Probenflüssigkeit geleitet wird. Dies gestattet wiederum eine sehr einfache Vorbehandlung, wobei eine räumliche und zeitliche Trennung der Vorbehandlung von der bevorzugten nachfolgenden Abtrennung von Bestandteilen der vorbehandelten Probenflüssigkeit einen besonders effizienten und/oder definierten Verfahrensablauf und/oder eine besonders effiziente Abtrennung von Bestandteilen aus der vorbehandelten Probenflüssigkeit gestattet.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. Es zeigt;
- Fig. 1: einen schematischen Schnitt einer vorschlagsgemäßen Vorrichtung gemäß einer ersten Ausführungsform;
- Fig. 2: eine Draufsicht der Vorrichtung gemäß Fig. 1;
- Fig. 3: einen schematischen Schnitt einer vorschlagsgemäßen Vorrichtung gemäß einer zweiten Ausführungsform mit geöffnetem Deckel;
- Fig. 4: einen schematischen Schnitt der Vorrichtung gemäß Fig. 3 mit geschlossenem Deckel;
- Fig. 5: einen schematischen Schnitt einer vorschlagsgemäßen Vorrichtung gemäß einer dritten Ausführungsform mit geöffnetem Deckel;
- Fig. 6: eine Draufsicht der Vorrichtung gemäß Fig. 5;
- Fig. 7: einen schematischen Schnitt der Vorrichtung gemäß Fig. 5 mit geschlossenem Deckel;
- Fig. 8: einen schematischen Schnitt einer vorschlagsgemäßen Vorrichtung gemäß einer vierten Ausführungsform; und
- Fig. 9: einen schematischen Schnitt einer vorschlagsgemäßen Vorrichtung gemäß einer fünften Ausführungsform.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

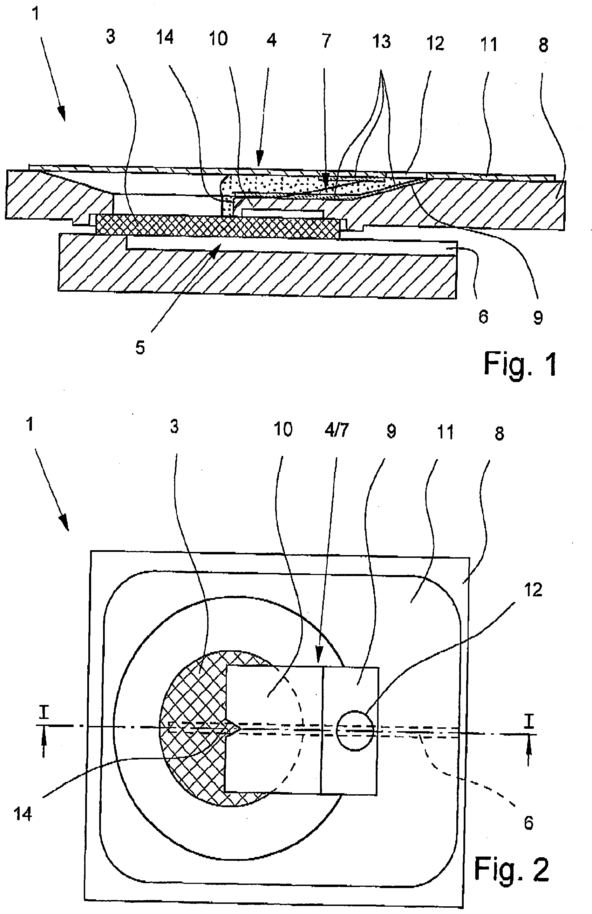

Fig. 1 zeigt in einem schematischen Schnitt eine erste, nicht beanspruchte Vorrichtung 1 zur Manipulation einer Probenflüssigkeit 2, vorzugsweise zur Abtrennung von Bestandteilen der Probenflüssigkeit 2, insbesondere Blut oder einer sonstigen menschlichen oder tierischen Körperflüssigkeit. Insbesondere dient die Vorrichtung 1 der Abtrennung von Blutbestandteilen, wie Blutzellen.

Vorzugsweise weist die Vorrichtung 1 zur Abtrennung von Bestandteilen eine Trenneinrichtung 3 auf, wie in Fig. 1 angedeutet. Die Trenneinrichtung 3 weist vorzugsweise eine Membran oder ein sonstiges geeignetes Filterelement auf. Die Membran bzw. das Filterelement kann bedarfsweise auch mehrlagig ausgebildet sein. Beispielsweise kann die Trenneinrichtung 3 bzw. die Membran wie in der WO 2009/106331 A2 beschrieben ausgebildet sein, die hiermit diesbezüglich als ergänzende Offenbarung eingeführt wird.

Die Vorrichtung 1 weist eine Aufnahmeeinrichtung 4 zur Aufnahme der Probenflüssigkeit 2 sowie vorzugsweise der Trenneinrichtung 3 nachgeordnete, insbesondere mikrofluidische Struktur 5 zur Ableitung der Probenflüssigkeit 2 nach der Abtrennung von Bestandteilen auf.

Das Aufnahmevolumen der Vorrichtung 1 oder einzelner Komponenten, wie der Aufnahmeeinrichtung 4, Trenneinrichtung 3 und/oder der Struktur 5, beträgt vorzugsweise weniger als 1 mm, insbesondere weniger als 500 µl, besonders bevorzugt weniger als 100 µl.

Die Aufnahmeeinrichtung 4, die Trenneinrichtung 3 und die Struktur 5 sind vorzugsweise übereinander angeordnet, insbesondere zumindest im Wesentlichen auf gegenüberliegenden Flachseiten der vorzugsweise flächigen Trenneinrichtung 3.

Beim Darstellungsbeispiel weist die Struktur 5 vorzugsweise einen Kanal 6 zur Ableitung von Probenflüssigkeit 2 nach dem Abtrennen von Bestandteilen durch die Trenneinrichtung 3 auf. Der Kanal 6 oder eine davon gebildete Kammer schließt sich insbesondere abströmseitig an die Trenneinrichtung 3 an und leitet die Probenflüssigkeit 2 beispielsweise an eine weitere Struktur, an einen Untersuchungsbereich oder dergleichen weiter, die bzw. der auch von der Vorrichtung 1 gebildet sein kann. Insbesondere kann die Vorrichtung 1 daher auch der Untersuchung und beispielsweise Bestimmung von Analyten oder sonstigen Werten der Probenflüssigkeit 2 dienen.

Die Aufnahmeeinrichtung 4 weist einen Aufnahmebereich 7 zum Dosieren, Anhalten und/oder Verzögern der Probenflüssigkeit 2, insbesondere durch Kapillarkraft auf Der Aufnahmebereich 7 ist vorzugsweise stromauf, über und/oder auf der Trenneinrichtung 3 angeordnet.

Alternativ ist es auch möglich, dass die Aufnahmeeinrichtung 4 bzw. der Aufnahmebereich 7 stromab oder unterhalb der Trenneinrichtung 3 angeordnet bzw. dieser nachgeordnet ist, In diesem Fall kann die Aufnahmeeinrichtung 4 bzw. der Aufnahmebereich 7 insbesondere die von der Trenneinrichtung 3 abgegebene, hier besonders bevorzugt gefilterte Probenflüssigkeit 2 zurückhalten, bis beispielsweise eine gewisse Menge an Probenflüssigkeit 2 von der Trenneinrichtung 3 separiert bzw, abgegeben wurde und/oder in der Aufnahmeeinrichtung 4 bzw. im Aufhahmebereich 7 gesammelt wurde. Die Probenflüssigkeit 2 kann danach beispielsweise an die sich anschließende Struktur 5 abgegeben werden.

Beim Darstellungsbeispiel weist die Vorrichtung 1, die Trenneinrichtung 3 bzw. die Aufnahmeeinrichtung 4 einen Träger 8 auf, der insbesondere die Trenneinrichtung 3 hält oder trägt und/oder der die Aufnahmeeinrichtung 4 bzw. deren Aufnahmebereich 7 vorzugsweise bildet, hält und/oder begrenzt. Jedoch sind auch andere konstruktive Lösungen möglich,

Der Träger 8 ist vorzugsweise plattenartig und/oder flach ausgebildet.

Der Aufnahmebereich 7 weist beim Darstellungsbeispiel vorzugsweise eine schräge Rampe 9 und/oder einen Leitbereich 10 auf. Beim Darstellungsbeispiel sind die Rampe 9 und der Leitbereich 10 vorzugsweise durch den Träger 8 gebildet oder von diesem getragen.

Der Aufhahmebereich 7 bzw. die Rampe 9 und/oder der Leitbereich 10 ist bzw. sind vorzugsweise oberhalb oder über der Trenneinrichtung 3 angeordnet und/oder erstrecken sich über diese, Beim Darstellungsbeispiel verläuft der Leitbereich 10 vorzugsweise im Wesentlichen beabstandet zu oder oberhalb der Trenneinrichtung 3 insbesondere ist der Leitbereich 10 plateauartig über der Trenneinrichtung 3 bzw. beabstandet zu dieser angeordnet. Vorzugsweise ist der Leitbereich 10 durch eine dem Aufnahmebereich 7 zugewandte, bei der Darstellung gemäß Fig. 1 nach oben weisende Wandung gebildet.

Der Aufnahmebereich 7 bzw, die Rampe 9 und/oder der Leitbereich 10 ist bzw, sind vorzugsweise - zumindest teilweise - durch eine zugeordnete Abdeckung 11 abgedeckt. Die Abdeckung 11 ist vorzugsweise folienartig ausgebildet und kann beispielsweise durch eine Klebefolie oder auflaminierte Folie oder dergleichen gebildet sein. Beim Darstellungsbeispiel ist die Abdeckung 7 vom Träger 8 gehalten. Jedoch sind auch andere konstruktive Lösungen möglich. Beispielsweise ist auch ein massiver Deckel als Abdeckung 7 einsetzbar.

Der Aufnahmebereich 7 ist vorzugsweise zwischen der Rampe 9 und/oder dem Leitbereich 10 einerseits und der Abdeckung 11 andererseits gebildet.

Zur Befüllung bzw. Aufnahme der Probenflüssigkeit 2 weist die Aufnahmeeinrichtung 4 bzw. die Abdeckung 11 vorzugsweise eine Aufnahmeöffnung 12 auf, die in Fig. 1 angedeutet ist.

Die Aufnahmeöffnung 12 ist vorzugsweise benachbart zu und insbesondere am oberen Ende bzw, Beginn der Rampe 9 angeordnet,

Der Aufnahmebereich 7 ist vorzugsweise derart gestaltet, dass beim Befüllen mit der Probenflüssigkeit 2 diese zunächst im Aufnahmebereich 7 angehalten oder verzögert wird. Dies wird beim Darstellungsbeispiel insbesondere dadurch erreicht, dass die Probenflüssigkeit 2 anfänglich sehr stark zwischen der Rampe 9 und der zugeordneten Abdeckung 11 gehalten wird und von dort aus langsam den Aufnahmebereich 7 füllt, bis der Leitbereich 10 zumindest im Wesentlichen mit der Probenflüssigkeit 2 befüllt ist bzw. bis die Flüssigkeitsfront bei der Darstellung in Fig, 1 den linken Endbereich des Aufnahmebereichs 7 erreicht, wie in Fig. 1 dargestellt.

Das Füllen des Aufnahmebereichs 7 mit der Probenflüssigkeit 2 ist für den Benutzer vorzugsweise sichtbar. Die Abdeckung 11 ist hierzu beispielsweise transparent ausgebildet. Wenn der Aufnahmebereich 7 gefüllt ist kann der Benutzer erkennen, dass ausreichend Probenflüssigkeit 2 aufgenommen wurde. Die Aufnahmeeinrichtung 4 bzw. deren Aufnahmebereich 7 dient also insbesondere auch einem einfachen Dosieren der Probenflüssigkeit 2.

Fig. 2 zeigt in einer schematischen Draufsicht die Vorrichtung 1, wobei die Abdeckung 11 transparent dargestellt ist und wobei aus Übersichtsgründen die Probenflüssigkeit 2 weggelassen wurde. Fig. 1 stellt einen Schnitt entlang Linie I - I von Fig. 2 dar.

Die Aufnahmeeinrichtung 4 oder deren Aufnahmebereich 7 weist vorzugsweise eine Chemikalie 13 zur Vorbehandlung der Probenflüssigkeit 2 vor der Zuführung zur Trenneinrichtung 3 auf Die Chemikalie 13 ist in Fig. 1 schematisch als Beschichtung dargestellt, die insbesondere im Aufnahmebereich 7 angeordnet ist, vorzugsweise auf der Rampe 9, dem Leitbereich 10 und/oder der Abdeckung 11, wie in Fig. 1 schematisch angedeutet.

Bei der Chemikalie 13 handelt es sich insbesondere um eine Trockenchemikalie bzw. eingetrocknete Chemikalie. Die Chemikalie 13 wird durch die Probenflüssigkeit 2 gelöst und kann dann zur Vorbehandlung mit der Probenflüssigkeit 2 reagieren. Dies erfolgt vorschlagsgemäß vor der Zuleitung der Probenflüssigkeit 2 zu der Trenneinrichtung 3.

Gemäße einer Variante enthält die Chemikalie 13 ein Agglutinationsmittel oder ist daraus gebildet. So kann eine Zusammenballung bzw. Agglomeration insbesondere von Blutzellen oder dergleichen bei der Aufnahme von Blut als Probenflüssigkeit 2 bewirkt oder unterstützt werden. Dies ist der dann nachfolgenden Abtrennung von Blutzellen in der Trenneinrichtung 3 zuträglich. Aus Versuchen ergibt sich, dass daraus ein wesentlicher besserer Durchsatz der Probenflüssigkeit 2 durch die Trenneinrichtung 3 erreichbar ist,

Gemäß einer anderen Variante enthält die Chemikalie 13 vorzugsweise ein gerinnungshemmendes Mittel oder sonstiges eine Deagglomeration bewirkendes oder förderndes Mittel oder ist daraus gebildet. Auch hierdurch lässt sich die nachfolgende Abtrennung von Bestandteilen, insbesondere Blutbestandteilen, aus der Probenflüssigkeit 2 in der Trenneinrichtung 3 positiv beeinflussen.

Nach der Vorbehandlung der Probenflüssigkeit 2 wird diese an die Trenneinrichtung 3 weitergeleitet. Dies erfolgt bei der dargestellten nicht beanspruchten Vorrichtung vorzugsweise durch eine fluidische Verbindung zwischen der Aufnahmeeinrichtung 4 bzw. dem Aufnahmebereich 7 einerseits und der Trenneinrichtung 3 andererseits. Die fluidische Verbindung ist beim Darstellungsbeispiel insbesondere als Drosselverbindung zur gedrosselten Leitung von Probenflüssigkeit 2 aus dem Aufnahmebereich 7 zur Trenneinrichtung 3 ausgebildet. Besonders bevorzugt ist die fluidische Verbindung durch einen Kanal, eine Kerbe 14 oder dergleichen gebildet, der bzw, die vorzugsweise den Aufnahbereich 7 bzw, Leitbereich 10 mit der Trenneinrichtung 3 verbindet, wie in Fig. 1 und 2 schematisch angedeutet.

Die fluidische Verbindung ist vorzugsweise an einem anderen Ende als die Aufnahmeöffnung 12 am Aufnahmebereich 7 angeordnet bzw. angeschlossen, um insbesondere erst nach zumindest im Wesentlichen vollständigem Füllen des Aufnahmebereichs 7 mit der Probenflüssigkeit 2 diese an die Trenneinrichtung 3 - vorzugsweise langsam bzw. gedrosselt - weiterzuleiten.

Die fluidische Verbindung führt beim Darstellungsbeispiel die in der Aufnahmeinrichtung 4 bzw, im Aufhahmebereich 7 vorzugsweise vorbehandelte Probenflüssigkeit 2 der Trenneinrichtung 3 aufnahmeseitig, insbesondere zumindest im Wesentlichen zentral zu. Bei Bedarf können auch mehrere fluidische Verbindungen zur Trenneinrichtung 3 gebildet sein, insbesondere um die Probenflüssigkeit 2 der Trenneinrichtung 3 an verschiedenen Stellen oder Bereichen zuzuführen.

Gemäß einer Variante ist es auch möglich, dass die fluidische Verbindung zunächst durch die Chemikalie 13 oder eine sonstige zusätzliche Chemikalie oder ein sogenanntes Zeitsteuerglied oder dergleichen zumindest temporär blockiert oder gesperrt wird, um eine bestimmte, gewisse oder vorbestimmte Vorbehandlungszeit der Probenflüssigkeit 2 in der Aufnahmeeinrichtung 4 bzw. im Aufnahmebereich 7 zu gewährleisten.

In Fig. 1 ist angedeutet, wie die Probenflüssigkeit 2 über die fluidische Verbindung aus dem Aufnahmebereich 7 bereits mittig - hier durch die Kerbe 14 - zu der Trenneinrichtung 3 strömt. Anschließend erfolgt in der Trenneinrichtung 3 das Abtrennen von Bestandteilen der Probenflüssigkeit 2, insbesondere von Blutzellen oder dergleichen. Hierbei fließt die Probenflüssigkeit 2 zumindest im Wesentlichen in Dickenrichtungen durch die Trenneinrichtung 3, wobei sich die Probenflüssigkeit 2 jedoch auf der Zuführungsseite der Trenneinrichtung 3 insbesondere auch flächig verteilt.

Nach der Abtrennung von Bestandteilen wird die Probenflüssigkeit 2 von der Struktur 5 bzw, deren Kanal 6 oder dergleichen aufgenommen und abgeleitet. Dies ist in Fig. 1 nicht dargestellt.

Die Probenflüssigkeit 2 wird im Aufnahmebereich 7 bzw. auf der Rampe 9 und insbesondere auf dem Leitbereich 10 vorzugsweise durch Kapillarkraft gegen ein undefiniertes, seitliches Abfließen auf die Trenneinrichtung 3 gehalten. Dies wird insbesondere beim Darstellungsbeispiel vorzugsweise durch einen verhältnismäßig geringen Abstand zu der Abdeckung 11, von beispielsweise weniger als 100 µm erreicht. Grundsätzlich sind jedoch auch andere Distanzen bzw. Abstände möglich, beispielsweise von mehreren Millimetern.

Nachfolgend werden weitere Vorrichtungen 1 anhand der weiteren Figuren erläutert, wobei lediglich wesentliche Unterschiede gegenüber des ersten Darstellungsbeispiels erläutert werden.

Die bisherigen Ausführungen und Erläuterungen gelten daher insbesondere ergänzend oder entsprechend, auch wenn eine wiederholte Beschreibung weggelassen ist.

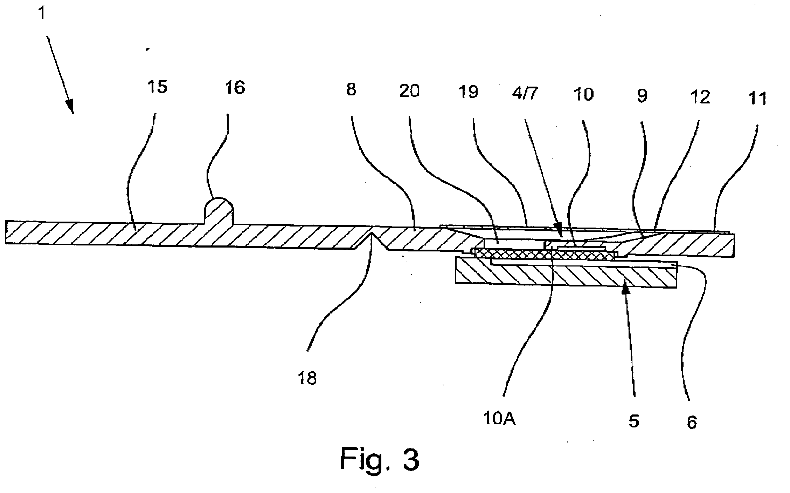

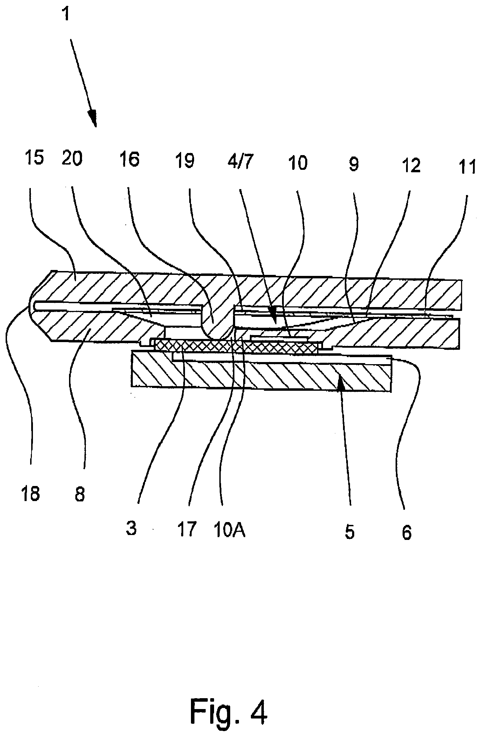

Fig. 3 zeigt in einem schematischen Schnitt eine zweite, nicht beanspruchte Vorrichtung 1 mit geöffnetem Deckel 15 und in Fig. 4 in einem schematischen Schnitt mit geschlossenem Deckel 15. In Fig. 3 und 4 sind weder die Probenflüssigkeit 2 noch die Chemikalie 13 aus Vereinfachungsgründen dargestellt.

Bei der zweiten Vorrichtung ist die mikrofluidische Verbindung zwischen dem Aufnahbereich 7 bzw. Leitbereich 10 einerseits und der Trenneinrichtung 3 andererseits gegenüber der ersten Vorrichtung geändert. Die fluidische Verbindung wird hier durch ein Leitelement 16 hergestellt. Das Leitelement 16 ist vorzugsweise als stiftartiger Vorsprung am Deckel 15 gebildet. Jedoch sind auch andere konstruktive Lösungen möglich.

Nach Füllen des Aufnahmebereichs 7 mit der Probenflüssigkeit 2 und bevorzugter Vorbehandlung durch Lösen der nicht dargestellten Chemikalie 13 wird die fluidische Verbindung zur Trenneinrichtung 3 durch Heranbewegen des Leitelements 16 hergestellt. Dies erfolgt beim Darstellungsbeispiel insbesondere dadurch, dass das Leitelement 16 über oder auf die Trenneinrichtung 3 bewegt bzw. geklappt wird und/oder in die Nähe des Aufnahmebereichs 7 bzw. Leitbereichs 10 gebracht wird. Besonders bevorzugt wird zwischen dem Leitelement 16 und dem Aufnahmebereich 7 bzw, dem angrenzenden Leitbereich 10 oder einer sich anschließenden Wandung 10A ein mikrofluidischer bzw. kapillarer Kanal 17 für die nicht dargestellte Probenflüssigkeit 2 zur Trenneinrichtung 3 hin gebildet, wie in Fig. 4 angedeutet, Bei der zweiten Ausführungsform bildet also der mikrofluidische bzw. kapillare Kanal 17 die fluidische Verbindung.

In beiden Ausführungsformen erfolgt die Leitung der Probenflüssigkeit 2 zur Trenneinrichtung 3 vorzugsweise zumindest primär durch Kapillarkräfte.

Bei der zweiten Vorrichtung ist der Deckel 15 vorzugsweise klappbar an der Vorrichtung 1 bzw. dem Träger 8 angebracht und vorzugsweise um ein Scharnier 18, das vorzugsweise als Filmscharnier ausgebildet ist, schwenkbar. Jedoch sind auch andere konstruktive Lösungen möglichen.

Das Herstellen bzw. Öffnen der fluidischen Verbindung erfolgt bei der zweiten Vorrichtung also insbesondere durch Umklappen bzw. Schließen des Deckels 15 bzw. der Vorrichtung 1.

Bei der zweiten Vorrichtung weist die Abdeckung 11 vorzugsweise eine (zweite) Durchbrechung 19 auf, damit das Leitelement 16 in den Aufnahmeraum 20 oberhalb der Trenneinrichtung 3 bzw. benachbart zum Aufnahmebereich 7 / Leitbereich 10 eingreifen kann. Jedoch sind auch andere konstruktive Lösungen möglichen.

Besonders bevorzugt ist der mikrofluidische bzw. kapillare Kanal 17 zwischen dem Leitelement 16 und der Wandung 10A gebildet, die sich - beispielsweise am Ende oder an der Kante des Leitbereichs 10 - zur Trenneinrichtung 3 hin erstreckt und gegebenenfalls auf dieser aufliegt.

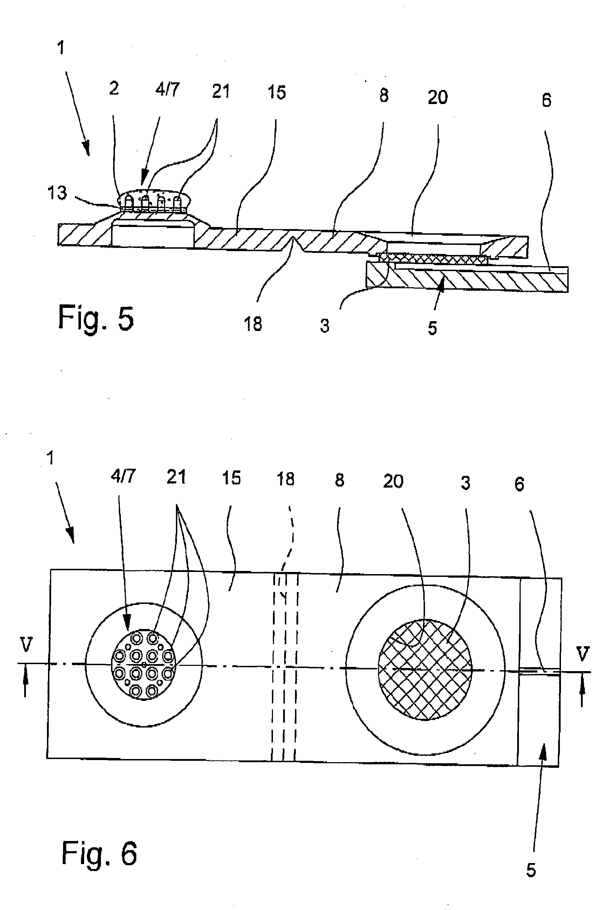

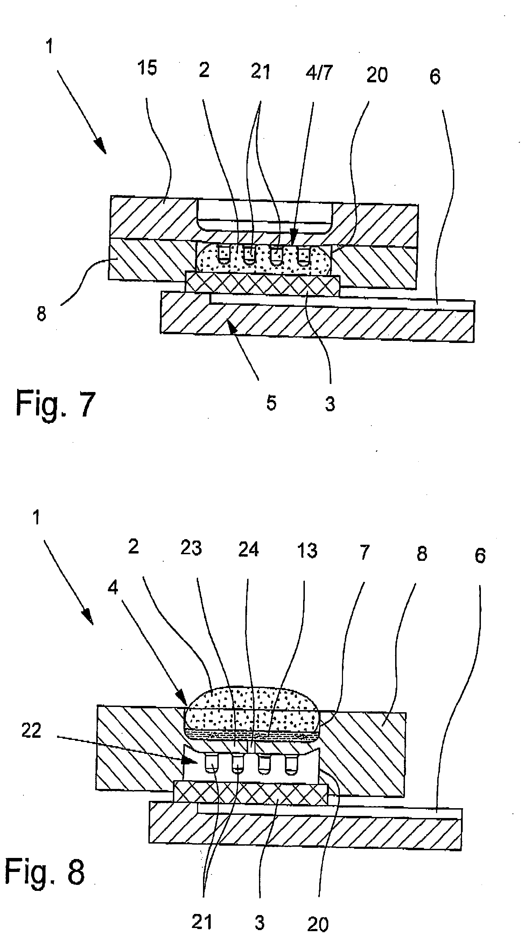

Fig. 5 zeigt in einem schematischen Schnitt eine dritte, nicht beanspruchte Vorrichtung 1 mit geöffnetem Deckel 15. Fig. 6 zeigt die Vorrichtung 1 in einer schematischen Draufsicht. Fig. 7 zeigt die Vorrichtung 1 bei geschlossenem Deckel 15 bzw. bei auf der Trenneinrichtung 1 angeordnetem Aufhahmebereich 7.

Bei der dritten Vorrichtung ist der Aufnahmebereich 7 über oder auf die Trenneinrichtung 3 bewegbar, insbesondere klappbar, um dadurch die Probenflüssigkeit 2 der Trenneinrichtung 3 zuzuleiten. Dies wird beim Darstellungsbeispiel insbesondere dadurch erreicht, dass die Aufnahmeeinrichtung 4 bzw. der Aufnahmebereich 7 vorzugsweise am Deckel 15 der Vorrichtung 1 angeordnet wird, der seinerseits, vorzugsweise klappbar, insbesondere aufgrund des Scharniers 18, über oder auf die Trenneinrichtung 3 bewegbar ist.

Die Aufnahmeeinrichtung 4 bzw. der Aufnahmebereich 7 weist hier vorzugsweise eine Halte- oder Dosierstruktur auf, die besonders bevorzugt aus einer Vielzahl von Erhebungen bzw. Säulen 21 gebildet ist. Die Erhebungen bzw. Säulen 21 sind insbesondere feldartig angeordnet bzw, bilden ein Säulenfeld. So wird eine bereichsweise Erhöhung der Kapillarität erreicht. Weiter kann so die Probenflüssigkeit 2 festgehalten werden. Insbesondere wird eine einfache Dosierung ermöglicht, da ein nicht dargestellter Benutzer erkennen kann, wenn die Erhebungen bzw. Säulen 21 - also das Säulenfeld - von der Probenflüssigkeit 2 bedeckt sind, wie in der Fig. 5 schematisch angedeutet,

Die Aufnahmeeinrichtung 4 bzw. der Aufnahmebereich 7 ist vorzugsweise mit der Chemikalie 13 versehen, wie in Fig. 5 angedeutet. Beispielsweise ist diese am Fuße der Erhebungen bzw. Säulen 21 angeordnet oder eingetrocknet. Jedoch sind auch andere lokale Anordnungen möglich.

Die Chemikalie 13 ist vorzugsweise sehr schnell, insbesondere innerhalb von 1 bis 2 Sekunden durch die Probenflüssigkeit 2 lösbar. Besonders bevorzugt ist hierzu weniger als 1 Sekunde erforderlich. Grundsätzlich kann das Vorbehandeln der Probenflüssigkeit 2 bzw. das Lösen der Chemikalie 13 auch langsamer erfolgen bzw, mehr Zeit benötigen, beispielsweise mehr als 5 Sekunden und/oder gegebenenfalls auch eine Minute oder mehr.

Nach dem Füllen des Aufnahmebereichs 7, also Dosieren der Probenflüssigkeit 2, und der bevorzugten Vorbehandlung durch die Chemikalie 13 wird der Aufnahmebereich 7 bzw, die vorbehandelte Probenflüssigkeit 2 mit der Trenneinrichtung 3 fluidisch in Kontakt gebracht, hier besonders bevorzugt durch Bewegen oder Klappen des Aufnahmebereichs 7 über oder auf die Trenneinrichtung 3, wie in Fig. 7 dargestellt.

Der Aufnahmebereich 7 bzw. die Probenflüssigkeit 2 wird also in den Aufnahmeraum 20 über der Trenneinrichtung 3 hineinbewegt und mit der Trenneinrichtung 3 fluidisch in Kontakt gebracht. Die Probenflüssigkeit 2 kann - insbesondere aufgrund von Kapillarkräften - durch die Trenneinrichtung 3 strömen und da durch kann die Abtrennung von Bestandteilen der Probenflüssigkeit 2 erfolgen.

Fig. 8 zeigt in einem schematischen Schnitt eine vierte, nicht beanspruchte Vorrichtung 1. Hier weist die Aufnahmeeinrichtung 4 zwischen dem Aufnahmebereich 7 und der Trenneinrichtung 3 zusätzlich einen Zwischenspeicher 22 auf, der beispielsweise oberhalb oder im Aufnahmeraum 20 der Trenneinrichtung 3 gebildet sein kann.

Bei der vierten Vorrichtung ist der Aufhahmebereich 7 beispielsweise durch eine Vertiefung im Träger 8 gebildet, Der Aufnahmebereich 7 ist vorzugsweise durch eine Zwischenwand 23 vom Zwischenspeicher 22 getrennt und/oder über eine fluidische Verbindung, vorzugsweise eine Drosselverbindung, die hier durch eine Bohrung 24 oder dergleichen gebildet ist, mit dem Zwischenspeicher 22 verbunden.

Der Zwischenspeicher 22 ist vorzugsweise mit einer Dosier- oder Haltestruktur, insbesondere aufgebaut aus einer Vielzahl von Erhebungen oder Säulen 21, versehen, wie in Fig. 8 angedeutet. Insbesondere ragen die Erhebungen bzw. Säulen 21 von der Zwischenwand 23 ab und/oder sind von dem Trennelement 3 etwas beabstandet. Insbesondere wird so erreicht, dass der Zwischenspeicher 22 erst nach zumindest weitgehend vollständigem Füllen mit Probenflüssigkeit 2 diese an die Trenneinrichtung 3 weiterleitet. Dies wird beim Darstellungsbeispiel dadurch erreicht, dass die aus dem Aufhahmebereich 7 durch die fluidische Verbindung bzw. Bohrung 24 in den Zwischenspeicher 22 strömende Probenflüssigkeit 2 dort zunächst zwischen den Säulen 21 aufgrund der herrschenden Kapillarkräfte gehalten wird und erst nach weitgehend vollständigem Füllen die Probenflüssigkeit 2 in fluidischen Kontakt mit der Trenneinrichtung 3 tritt. So kann zusätzliche Zeit für die Vorbehandlung für die Probenflüssigkeit 2 vor Zuleitung zur Trenneinrichtung 3 gewonnen oder gewährleistet werden.

Die Chemikalie 13 ist beim Darstellungsbeispiel im Aufhahmebereich 7 angeordnet, kann alternativ oder zusätzlich jedoch auch im Zwischenspeicher 22 angeordnet sein.

Wie in Fig. 8 schematisch angedeutet, kann die Chemikalie 13 bedarfsweise die fluidische Verbindung - hier die Bohrung 24 - zur Ableitung der Probenflüssigkeit 2 aus dem Aufnahmebereich 7 überdecken oder blockieren. So kann erreicht werden, dass die Probenflüssigkeit 2 zunächst die Chemikalie 13 löst, bevor die Probenflüssigkeit 2 weiter - gegebenenfalls über den Zwischenspeicher 22 - zur Trenneinrichtung 3 strömen kann.

Die bei der vierten Vorrichtung besonders bevorzugte schalenartige Vertiefung des Aufnahmebereichs 7 ermöglicht wiederum eine einfache Dosierung der Probenflüssigkeit 2. Ein nicht dargestellter Benutzer kann nämlich sehr leicht erkennen, dass nach vollständigem Füllen des Aufnahmebereichs 7 ausreichend Probenflüssigkeit 2 zugegeben wurde. Dies gestattet also eine einfache Dosierung.

Bei der vierten Vorrichtung sind die Trenneinrichtung 3, der Zwischenspeicher 22 und/oder der Aufnahmebereich 7 vorzugsweise übereinander angeordnet.

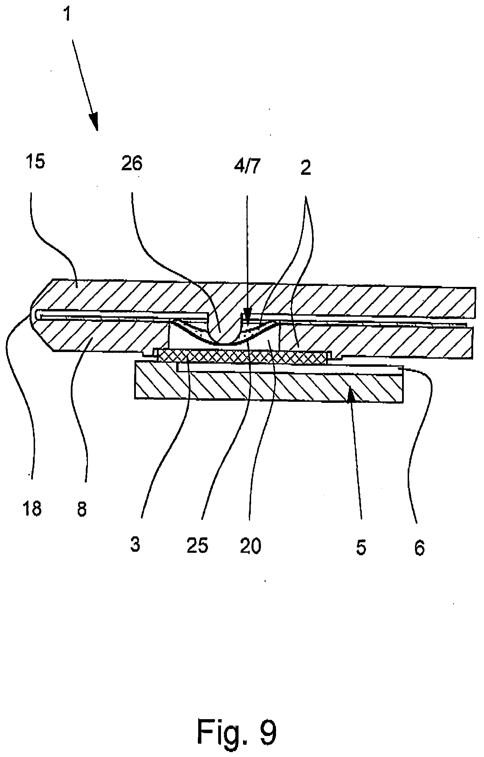

Fig. 9 zeigt in einem schematischen Schnitt eine Vorrichtung 1 gemäß einer beanspruchten Ausführungsform. Die Aufnahmeeinrichtung 4 bzw. der Aufnahmebereich 7 weist hier einen verformbaren Verformungsbereich auf, der durch eine gelochte Folie, Membran 25 oder dergleichen gebildet ist. Die Membran 25 ist vorzugsweise am Träger 8 gehalten, beispielsweise mit einer bestimmten Vorspannung.

Die Aufnahmeeinrichtung 4 bzw. der Aufnahmebereich 7 und/oder der Verformungsbereich bzw. die Membran 25 kann mit der Chemikalie 13 versehen sein, die hier aus Vereinfachungsgründen nicht dargestellt ist. Nach Füllen des Aufnahmebereichs 7 mit der Probenflüssigkeit 2 und entsprechender Vorbehandlung durch Lösen der Chemikalie 13 kann der Verformungsbereich bzw. die Membran 25 durch Verformen mit der Trenneinrichtung 3 fluidisch in Kontakt gebracht werden, wie in Fig. 9 angedeutet. Dies erfolgt mittels eines Manipulierelements 26, das in den Aufnahmebereich 7 bzw. Aufnahmeraum 20 bzw. zur Trenneinrichtung 3 hin bewegbar ist, durch Umklappen des Deckels 15, der das Manipulierelement 26 trägt, wie in Fig. 9 angedeutet.

Durch das Verformen des Verformungsabschnitts bzw. der Membran 25 wird die Probenflüssigkeit 2 fluidisch mit der Trenneinrichtung 3 in Kontakt gebracht und kann auf und durch die Trenneinrichtung 3 strömen.

Es ist anzumerken, dass einzelne Merkmale und Aspekte der verschiedenen Vorrichtungen beliebig miteinander kombiniert, aber auch unabhängig voneinander realisiert werden können.

### Bezugszeichenliste:

- 1: Vorrichtung
- 2: Probenflüssigkeit
- 3: Trenneinrichtung
- 4: Aufhahmeeinrichtung
- 5: Struktur
- 6: Kanal
- 7: Aufnahmebereich
- 8: Träger
- 9: Rampe
- 10: Leitbereich
- 10A: Wandung
- 11: Abdeckung
- 12: Aufnahmeöffnung
- 13: Chemikalie
- 14: Kerbe
- 15: Deckel
- 16: Leitelement
- 17: kapillarer Kanal
- 18: Scharnier
- 19: Durchbrechung
- 20: Aufnahmeraum
- 21: Säule
- 22: Zwischenspeicher
- 23: Zwischenwand
- 24: Bohrung
- 25: Membran
- 26: Manipulierelement

## Patentansprüche

1. Mikrofluidische Vorrichtung (1) zur Abtrennung von Bestandteilen einer Probenflüssigkeit (2), insbesondere Blut,
mit einer Aufnahmeeinrichtung (4) zur Aufnahme der Probenflüssigkeit (2),
mit einer Trenneinrichtung (3) zur Abtrennung von Bestandteilen der Probenflüssigkeit (2), wobei die Probenflüssigkeit (2) der Trenneinrichtung (3) aus der Aufnahmeeinrichtung (4) zuführbar ist, und
mit einer der Trenneinrichtung (3) nachgeordneten Struktur (5) zur Ableitung der Probenflüssigkeit (2) nach der Abtrennung von Bestandteilen,
**dadurch gekennzeichnet,**
**dass** die Aufnahmeeinrichtung (4) einen Aufnahmebereich (7) zum Anhalten oder Verzögern der Probenflüssigkeit (2) durch Kapillarkraft vor Zuführung zu der Trenneinrichtung (3) aufweist,
**dass** der Aufnahmebereich (7) einen durch eine gelochte Folie oder eine Membran (25) gebildeten Verformungsbereich aufweist, der durch Verformen mit der Trenneinrichtung (3) fluidisch in Kontakt bringbar ist, wobei durch das Verformen die Probenflüssigkeit (2) auf und durch die Trenneinrichtung (3) strömen kann, und
**dass** die Vorrichtung (1) einen Deckel (15) aufweist, der ein Manipulierelement (26) trägt, wobei das Manipulierelement (26) durch Umklappen des Deckels (15) in den Aufnahmebereich (7) bewegbar ist, um den Verformungsbereich zu verformen.

2. Vorrichtung nach Anspruch 1, **dadurch gekennzeichnet, dass** die Aufnahmeeinrichtung (4) eine von der Probenflüssigkeit (2) lösbare Chemikalie (13) zur Vorbehandlung der Probenflüssigkeit (2) vor der Zuführung zur Trenneinrichtung (3) aufweist.

3. Vorrichtung nach Anspruch 2, **dadurch gekennzeichnet, dass** die Chemikalie (13) ein Agglutinationsmittel enthält oder ist.

4. Vorrichtung nach Anspruch 2, **dadurch gekennzeichnet, dass** die Chemikalie (13) ein gerinnungshemmendes Mittel enthält oder ist.

5. Verfahren zur Abtrennung von Bestandteilen einer Probenflüssigkeit (2), insbesondere Blut,
wobei die Probenflüssigkeit (2) in eine mikrofluidische Vorrichtung (1) aufgenommen wird und dort in einem Aufnahmebereich (7) der Vorrichtung (1) getrennt von einer Trenneinrichtung (3) der Vorrichtung (1) mit einer Chemikalie (13) vorbehandelt wird,
wobei ein Deckel (15) der Vorrichtung (1) umgeklappt wird, so dass ein Manipulierelement (26), das der Deckel (15) trägt, in den Aufnahmebereich (7) bewegt wird und einen Verformungsbereich einer im Aufnahmebereich (7) angeordneten Membran (25) oder gelochten Folie verformt,
wobei durch das Verformen des Verformungsbereichs ein fluidischer Kontakt zur Trenneinrichtung (3) hergestellt wird, so dass die Probenflüssigkeit (2) nach der Vorbehandlung durch die Trenneinrichtung (3) zur Abtrennung von Bestandteilen aus der vorbehandelten Probenflüssigkeit (2) geleitet wird.

## Claims

1. Microfluidic apparatus (1) for separating components of a sample liquid (2), in particular blood,
with a receiving device (4) for receiving the sample liquid (2),
with a separating device (3) for separating components of the sample liquid (2), wherein the sample liquid (2) is feedable to the separating device (3) from the receiving device (4), and
with a structure (5) downstream of the separating device (3) for discharging the sample liquid (2) after the separation of components,
**characterized**
**in that** the receiving device (4) has a receiving region (7) for stopping or retarding the sample liquid (2) by capillary force before feeding to the separating device (3),
**in that** the receiving region (7) has a deformation region which is formed by a perforated film or a membrane (25) and can be brought into fluidic contact with the separating device (3) by deformation, the sample liquid (2) being able to flow on and through the separating device (3) by the deformation, and
**in that** the apparatus (1) has a lid (15) which carries a manipulating element (26), the manipulating element (26) being movable into the receiving region (7) by turning over the lid (15) in order to deform the deformation region.

2. Apparatus according to claim 1, **characterized in that** the receiving device (4) comprises a chemical (13), which is soluble by the sample liquid (2), for pretreating the sample liquid (2) before feeding to the separating device (3).

3. Apparatus according to claim 2, **characterized in that** the chemical (13) contains or is an agglutinating agent.

4. Apparatus according to claim 2, **characterized in that** the chemical (13) contains or is an anticoagulant.

5. Method for separating components of a sample liquid (2), in particular blood,
wherein the sample liquid (2) is received in a microfluidic apparatus (1) and is pretreated there with a chemical (13) in a receiving region (7) of the apparatus (1) separated from a separating device (3) of the apparatus (1),
wherein a lid (15) of the apparatus (1) is turned over so that a manipulating element (26), which is carried by the lid (15), is moved into the receiving region (7) and deforms a deformation region of a membrane (25) or perforated film arranged in the receiving region (7),
wherein a fluidic contact to the separating device (3) is established by the deformation of the deformation region, so that the sample liquid (2) after the pretreatment is passed through the separating device (3) for the separation of components from the pretreated sample liquid (2).

## Revendications

1. Appareil (1) microfluidique destiné à la séparation des composants d'un liquide d'échantillon (2), en particulier du sang,
comprenant un dispositif de réception (4) destiné à la réception du liquide d'échantillon (2),
comprenant un dispositif de séparation (3) destiné à la séparation des composants du liquide d'échantillon (2), le liquide d'échantillon (2) pouvant être amené dans le dispositif de séparation (3) depuis le dispositif de réception (4) et
comprenant une structure (5) en aval du dispositif de séparation (3) destinée à l'évacuation du liquide d'échantillon (2) après la séparation des composants,
**caractérisé**
**en ce que** le dispositif de réception (4) comporte une zone de réception (7) pour arrêter ou décélérer le liquide d'échantillon (2) par force capillaire avant l'amenée au dispositif de séparation (3),
**en ce que** la zone de réception (7) comporte une zone de déformation formée par une feuille perforée ou une membrane (25), la zone de déformation pouvant être mise en contact fluidique avec le dispositif de séparation (3) par déformation, le liquide d'échantillon (2) pouvant s'écouler sur et à travers le dispositif de séparation (3) en raison de la déformation et
**en ce que** l'appareil (1) comporte un couvercle (15), lequel porte un élément de manipulation (26), l'élément de manipulation (26) étant mobile dans la zone de réception (7) en rabattant le couvercle (15) afin de déformer la zone de déformation.

2. Appareil selon la revendication 1, **caractérisé en ce que** le dispositif de réception (4) comporte un produit chimique (13) soluble par le liquide d'échantillon (2) pour prétraiter le liquide d'échantillon (2) avant l'amenée dans le dispositif de séparation (3).

3. Appareil selon la revendication 2, **caractérisé en ce que** le produit chimique (13) contient ou est un agent d'agglutination.

4. Appareil selon la revendication 2, **caractérisé en ce que** le produit chimique (13) contient ou est un anticoagulant.

5. Procédé pour la séparation des composants d'un liquide d'échantillon (2), en particulier du sang,
le liquide d'échantillon (2) étant reçu dans un appareil (1) microfluidique et prétraité avec un produit chimique (13) dans une zone de réception (7) du appareil (1), séparée d'un dispositif de séparation (3) du appareil (1),
un couvercle (15) du appareil (1) étant rabattu de telle sorte qu'un élément de manipulation (26), porté par le couvercle (15), soit déplacé dans la zone de réception (7) et déforme une zone de déformation d'une feuille perforée ou d'une membrane (25) disposée dans la zone de réception (7),
par la déformation de la zone de déformation, un contact fluidique avec le dispositif de séparation (3) étant établi de telle sorte que le liquide d'échantillon (2), après le prétraitement, soit guidé à travers le dispositif de séparation (3) pour séparer les composants du liquide d'échantillon (2) prétraité.
